# EUROPEAN PATENT SPECIFICATION

(11) **EP 2 441 514 B1**
(45) Date of publication and mention of the grant of the patent: **09.04.2014**
(21) Application number: 10786221.1
(22) Date of filing: 10.06.2010
(51) Int. Cl.: B01J 23/26, B01J 23/20, B01J 23/28, B01J 27/198, B01J 27/224, C07C 253/28, C07C 255/51, C07B 61/00

(54) **AMMOXIDATION CATALYST AND METHOD FOR PRODUCING NITRILE COMPOUND USING THE SAME**
AMMOXIDIERUNGSKATALYSATOR UND VERFAHREN ZUR HERSTELLUNG EINER NITRILVERBINDUNG DAMIT
CATALYSEUR D'AMMOXYDATION ET PROCÉDÉ DE FABRICATION D'UN COMPOSÉ DE NITRILE L'UTILISANT

(30) Priority: 11.06.2009 JP 2009140196
(43) Date of publication of application: 18.04.2012
(73) Proprietor: Mitsubishi Gas Chemical Company, Inc., Tokyo 100-8324 (JP)
(72) Inventor: YAMAMOTO, Kazunari, Niigata-shi Niigata 950-3112 (JP); KYUUKO, Youichi, Niigata-shi Niigata 950-3112 (JP); OKAMOTO, Atsushi, Niigata-shi Niigata 950-3112 (JP)
(74) Representative: HOFFMANN EITLE
(86) International application number: PCT/JP2010/059850
(87) International publication number: WO 2010/143690

(56) References cited:
- EP-A2- 2 135 671
- JP-A- 7 285 923
- JP-A- 11 033 399
- JP-A- 11 310 562
- JP-A- 2001 062 303

## Description

### Technical Field

The present invention relates to a process four producing a nitrile compound wherein a catalyst used for an ammoxidation reaction (reaction in which an organic compound is reacted with a mixed gas containing ammonia and oxygen) is used. Nitrile compounds are useful as raw materials for producing synthetic resins, agricultural chemicals and the like and intermediate raw materials for amines, isocyanates and the like.

### Background Art

Various catalysts used for ammoxidation reaction (hereinafter sometimes referred to as "ammoxidation catalyst" or merely "catalyst") are proposed, and publicly known are catalysts containing oxides of fifth group transition elements in the periodic table (particularly vanadium pentaoxide) and oxides of fourth group, sixth group and eighth group transition elements in the periodic table.

There is a report that an antimony-tellurium-containing metal oxide catalyst is used in a process for producing acrylonitrile by subjecting a lower hydrocarbon compound or a lower oxygen-containing organic compound to ammoxidation reaction (PTL 1).

Examples of ammoxidation catalysts used in a process for producing a nitrile compound by subjecting a carbocyclic compound having a side chain functional group to ammoxidation reaction include an alumina-molybdenum-based catalyst used in producing benzonitrile from toluene (PTL 2), a vanadium oxide-antimony oxide-potassium oxide catalyst used in producing o-phthalonitrile from o-xylene (PTL 3), a vanadium oxide-chromium oxide-molybdenum oxide catalyst used in producing isophthalonitrile from meta-xylene (PTL 4), a vanadium-potassium-antimony-titanium catalyst used in producing 2,6-dicyanonaphthalene from 2,6-dimethylnaphthalene (PTL 5), a vanadium-antimony-based catalyst used in producing dicyanoaniline from dimethylaniline (PTL 6) and the like.

Examples of ammoxidation catalysts used in a process for producing a nitrile compound by subjecting a heterocyclic compound having a side chain functional group to ammoxidation reaction include an iron-antimony-vanadium-containing catalyst used in producing 4-cyanopyridine from 4-methylpyridine (PTL 7), a molybdenum-phosphorus-based catalyst used in producing 2-cyanopyrazine from 2-methylpyrazine (PTL 8), a vanadium-phosphorus-silica catalyst used in producing 2-amino-3-cyanopyridine from 2-amino-3-methylpyridine (PTL 9), a vanadium-molybdenum-chromium-phosphorus-alumina catalyst used in producing 2,6-dichlorobenzonitrile from 2,6-dichlorotoluene (PTL 10) and the like.

Descriptions relating to a catalyst composition containing diamond are not found in publicly known documents regarding processes for producing nitrile compounds by subjecting organic compounds to ammoxidation reaction with a mixed gas containing ammonia and oxygen in the presence of a catalyst.

Catalysts described in the publicly known documents are improved in a catalyst activity, a reaction selectivity, a performance stability and the like in various manners and have reached a certain technical level, but more excellent catalysts are continuously desired to be developed. In particular, the reaction selectivity is desired to be enhanced, and since catalyst additive components are usually investigated in order to improve it, catalysts having a higher selectivity tend to have a larger number of components. In these catalysts, it is listed as problems to be improved that the catalyst preparation procedures such as preparing raw materials for the many components, pre-treatment of the many components and the like are cumbersome and complicated and that a catalyst composition range and a reaction condition range for providing an optimum reaction result are narrowed. Accordingly, it is useful to find out an additive component which can be easily used and have broad ranges of application regarding catalyst preparation conditions and reaction conditions.

A big reason for making it difficult to enhance the reaction selectivity is that a reaction in which organic compounds are subjected to ammoxidation reaction with a mixed gas containing ammonia and oxygen is a heavy exothermic reaction accompanied by a complete combustion reaction.

Accordingly, in order to improve the selectivity, an efficiency of removing heat has so far been tried to be enhanced by improving the reaction form, for example, using a fluid bed reactor. However, catalysts used for a fluid bed reactor are fine powders, and therefore changes such as dispersal and abrasion are caused during handling them to bring about deterioration of the selectivity thereof.

On the other hand, when a fixed bed reaction form is used, there is a disadvantage that since a part of a catalyst layer reaches a high temperature, it is difficult to control the reaction. Multilayer filling may be employed in order to solve this disadvantage, but it provides another disadvantage that labor and time for filling are required.

### Citation List

### Patent Literature

PTL 1: JP-A-63-209755
PTL 2: JP-B-44-17376
PTL 3: JP-A-2008-69156
PTL 4: JP-B-45-19051
PTL 5: JP-A-7-285923
PTL 6: JP-A-6-279388
PTL 7: JP-A-8-295672
PTL 8: JP-A-8-81448
PTL 9: JP-A-4-364169
PTL 10: JP-A-3-44362

### Summary of Invention

### Technical Problem

An object of the present invention is to solve the foregoing problems on conventional techniques, and provide a process for producing a nitrile compound at a high yield by using an ammoxidation catalyst for an efficient ammoxidation reaction of organic compounds with a mixed gas containing ammonia and oxygen.

### Solution to Problem

Intense investigations repeated by the present inventors on catalysts used in ammoxidation reaction of organic compounds with a mixed gas containing ammonia and oxygen have resulted in finding that a catalyst obtained by effectively combining vanadium oxide, titanium oxide and diamond enables an efficient ammoxidation reaction of organic compounds with a mixed gas containing ammonia and oxygen and enables a production of nitrile compounds at a high yield. Thus, they have reached the present invention.

That is, the present invention relates to a process for producing a nitrile compound including an ammoxidation reaction step in which an organic compound is reacted in a gas phase with a mixed gas containing ammonia and oxygen under the presence of an ammoxidation catalyst containing vanadium oxide, titanium oxide and diamond, wherein the diamond content is 0.1 70 % by weight based on the weight of the ammoxidation catalyst.

### Advantageous Effects of Invention

The yield and selectivity in the ammoxidation reaction can be enhanced by use of the ammoxidation catalyst employed in the present invention. In production of a nitrile compound, the yield of the nitrile compound can be enhanced by use of the ammoxidation catalyst.

### Description of Embodiments

### [Ammoxidation catalyst]

The ammoxidation catalyst employed in the present invention contains vanadium oxide, titanium oxide and diamond, and the diamond content is 0.1 - 70 % by weight based on the weight of the ammoxidation catalyst.

### (Diamond)

The ammoxidation catalyst employed in the present invention contains diamond. The diamond is not specifically restricted, and it may be a naturally produced product (natural product) or an artificially synthesized product (artificial product), and the natural product is preferred. In a case of the natural product, a production area and a grade thereof are not specifically restricted. In a case of the artificial product, it may be synthesized by methods which have so far been publicly known, and starting materials and methods thereof are not specifically restricted.

Publicly known as a synthetic method of the diamond are, for example, a method in which diamond is synthesized from graphite in the high temperature and high pressure conditions of 1500°C and 5 GPa under the presence of a transition metal such as cobalt, nickel, chromium, manganese, and tantalum; a method in which diamond is synthesized from graphite in the higher temperature and higher pressure conditions of 2000°C and 7 GPa under the presence of carbonates, hydroxides and sulfates of alkali metals or alkaline earth metals; a method in which diamond is obtained by subjecting graphite to direct phase transition in the further higher temperature and further higher pressure conditions of 3000°C and 15 GPa in the absence of a catalyst (static high pressure method); a method in which diamond is grown from a gas phase in a gas flow of a carbon-containing compound such as methane and carbon monoxide and a hydrogen by a microwave, a high frequency wave, heating and the like (chemical vapor deposition method); and a method in which diamond is synthesized by compressing carbonaceous substances by explosion of powder (explosion impact method). Monocrystalline or polycrystalline diamonds obtained by the above methods can be used.

A form of the diamond used in the present invention is not specifically restricted, and granular, tabular and flaky diamonds can be used. In a case of granular diamond, a particle size thereof is not specifically restricted as long as it can be used for producing catalysts together with other materials. However, diamonds of a mm size in either natural products or artificial products are very expensive for use as catalyst materials, and therefore powdery diamonds supplied at a lower cost are preferably used. Products obtained by crushing diamonds of a large size, origins of which may be either natural products or artificial products, can be used, and products naturally produced or synthesized in the form of powdery diamond can be used. Among them, fine powdery diamonds which are widely used as a polishing material are suitable because they have been subjected to classification treatment, chemical treatment and the like according to intended uses for polish and therefore they can be readily obtained as commercially available industrial products which are controlled in particle size distribution and amount of impurities. A particle diameter of the diamond is preferably 100 µm or less, more preferably 10 µm or less and further preferably 1 µm or less from the viewpoint that the diamond and oxide components (vanadium oxide, titanium oxide and, if necessary, other oxides) in the catalyst can be sufficiently dispersed and mixed to obtain the good catalyst. A lower limit of the particle diameter is not specifically restricted, and therefore the particle diameter is more than 0 µm, preferably 0.001 µm or more. As described above, fine powdery diamonds for a polishing material which are controlled in particle size distribution can be readily obtained, and therefore, for example, diamonds having been subjected to classification treatment using a sieve having a pore size (size of a mesh) of 100 µm can be used as the fine powdery diamonds having a particle diameter of 100 µm or less. An average particle diameter of the diamond is preferably 10 µm or less, more preferably 1 µm or less and further preferably 0.1 µm or less. In this connection, a particle diameter of the diamond can be readily measured and evaluated by means of an existing laser dispersion type particle size measuring equipment.

A purity of the diamond used in the present invention is not specifically restricted as well. It is known well that in both of the natural products and the artificial products, elements other than carbon originating in atmosphere in which the diamonds are formed and subsequent processing treatments are introduced into an outer surface and an inside of the diamonds and that they reach several thousand ppm in a certain case. A higher purity of the diamond is desirable, but if it is not the case, no problems shall be brought about on use thereof. Classification of Field (The Properties of Diamond, p. 641, Academic Press (1979)) is known well as a method for classifying diamonds by impurities contained therein and a color tone thereof, and any type of Ia type, Ib type, IIa type and IIb type which are referred to herein can be used alone or in a mixture.

In the present invention, an ammoxidation reaction of organic compounds is carried out under the coexistence of an ammonia and an oxygen-containing gas. Diamonds are less oxidizable as compared with organic compounds, but it doesn't mean that they are completely non-oxidizable. Diamonds having a high oxidation resistance are more preferably used for the purpose of avoiding loss of diamond by complete oxidation.

An oxidation resistance of diamond is considered to be influenced by the amounts of boron and transition metal elements contained therein and an oxidative functional group on a surface of the diamond. It is considered from the above matter that transition metal elements such as Fe, Co and Ni which are contained therein as impurities have a catalytic action of graphitizing the diamond at an atmospheric pressure and that an oxidation resistance of graphitized carbon is notably reduced, and therefore transition metal elements are contained therein preferably in smaller amounts. The examples of such a diamond include natural diamonds, diamonds synthesized under the presence of carbonates, hydroxides and sulfates of alkali metals or alkaline earth metals (JP-A-7-45652 and New Diamond, 15 (2), p. 13 to 19

(1999)) and diamonds synthesized by direct phase transition from graphite in the absence of a catalyst. Further, it is known as well that the oxidation resistance can be improved by enhancing a content of boron, and boron-containing diamonds (JP-A-2006-502955, US 2004/0018137 and etc.) can be used as well. Also, publicly known as well are methods for removing impurities such as transition metal elements contained in a synthesizing or processing step of diamonds (JP-A-63-303806, JP-A-9-25110, JP-A-9-328307 and etc.) and methods for hydrogenating a surface of diamond (The Chemical Society of Japan report, No. 11, p. 631 to 635 (2001) and etc.). Diamonds having been subjected to these treatments can be suitably used as well.

### (Vanadium oxide)

The ammoxidation catalyst employed in the present invention contains vanadium oxide. A valence of vanadium is not restricted as long as it falls in a range which can be accepted by vanadium, and vanadium oxide having a high valence is preferred in order to maintain stability under oxidative atmosphere and exert a catalytic performance. Examples thereof include VO₂ and V₂O₅, and VO₂ is preferred. At least a part of vanadium oxide may have a more complicated valence and a more complicated crystal phase such as V₃O₇, V₄O₉ and V₆O₁₃ under the reaction. Starting materials thereof are not specifically restricted as long as they provide oxides, and, for example, hydroxides, oxyammonium salts, chlorides, oxychlorides, oxynitrates, oxalates and oxyoxalates can be used. The above vanadium oxides can be used alone or in combination of two or more kinds thereof.

### (Titanium oxide)

The ammoxidation catalyst employed in the present invention contains titanium oxide. A valence of titanium is not restricted as long as it falls in a range which can be accepted by titanium, and titanium oxide having a high valence is preferred in order to maintain stability under oxidative atmosphere and exert a catalytic performance. TiO₂ is preferred as an example thereof. At least a part of titanium oxide may have a more complicated valence and a more complicated crystal phase under the reaction. Starting materials thereof are not specifically restricted as long as they provide oxides, and, for example, hydroxides, oxyammonium salts, chlorides, oxychlorides, oxynitrates, oxalates and oxyoxalates can be used. The above titanium oxides can be used alone or in combination of two or more kinds thereof.

### (Oxide A)

The ammoxidation catalyst employed in the present invention preferably further contains, as an oxide other than vanadium oxide and titanium oxide, at least one oxide (herein referred to as the oxide A) selected from the group consisting of chromium oxide, molybdenum oxide, tungsten oxide, zirconium oxide, iron oxide, cobalt oxide, nickel oxide, niobium oxide, boron oxide, aluminum oxide, germanium oxide, phosphorus oxide, antimony oxide and bismuth oxide. As the oxide A. chromium oxide, molybdenum oxide, iron oxide, boron oxide and phosphorus oxide are more preferred, and chromium oxide, molybdenum oxide, boron oxide and phosphorus oxide are further preferred. Complete combustion is suppressed by containing the oxide A as compared with a case of vanadium oxide alone, and the yield of the nitrile compound as the ammoxidation reaction product is increased.

The oxide A is not restricted in a valence in ranges which can be accepted by the respective elements, and the oxides having a high valence are preferred in order to maintain stability under oxidative atmosphere and exert a catalytic performance. Examples thereof include Cr₂O₃, CrO₂, CrO₃, MoO₂, MoO₃, WO₂, WO₃, ZrO₂, Fe₂O₃, Fe₃O₄, FeO, CoO, Co₃O₄, NiO, NbO₂, Nb₂O₅, B₂O₃, Al₂O₃, GeO₂, P₂O₅, Sb₂O₄, Sb₂O₅, Bi₂O₄ and Bi₂O₅, and Cr₂O₃, MoO₃, P₂O₅ and B₂O₃ are preferred. At least a part of the oxide A may have a more complicated valence and a more complicated crystal phase such as Mo₄O₁₁, Mo₈O₂₃, Mo₉O₂₆, W₁₈O₄₉ and W₂₀O₅₈ under the reaction. Starting materials thereof are not specifically restricted as long as they provide oxides, and, for example, hydroxide, chlorides, oxychlorides, oxyammonium salts, nitrates, acetates and oxalates can be used. The above oxides A can be used alone or in combination of two or more kinds thereof.

### (Oxide B)

The ammoxidation catalyst employed in the present invention may further contain, as an oxide other than vanadium oxide and titanium oxide, at least one oxide (herein referred to as the oxide B) selected from the group consisting of lithium oxide, sodium oxide, potassium oxide, rubidium oxide, cesium oxide, gallium oxide, silicon oxide, tin oxide, lead oxide, selenium oxide and tellurium oxide to the extent that the effects of the present invention are not damaged. The oxide B is not restricted in a valence in ranges which can be accepted by the respective elements, and the oxides having a high valence are preferred in order to maintain stability under oxidative atmosphere. Examples thereof include Li₂O, Na₂O, K₂O, Rb₂O, Cs₂O, Ga₂O₃, Si₂O, SnO₂, PbO₂, TeO₂, TeO₃ and SeO₂. Starting materials thereof are not specifically restricted as long as they provide oxides, and, for example, hydroxides, chlorides, nitrates, oxalates and carbonates can be used. The above oxides B can be used alone or in combination of two or more kinds thereof.

The oxides used in the present invention may be amorphous or crystal respectively, and when they have plural crystal structures in the same chemical composition, they may be used in a state of any one of them or a mixture of two or more of them. In a case of, for example, titanium oxide, the crystal forms of a rutile type, an anatase type and a brookite type in a chemical composition formula TiO₂ are known well, and it may be any one of them or a mixed crystal, a twin crystal or a mixture of two or more crystal forms selected from them.

In the present invention, the respective oxides in the catalyst may be a mixture of the respective components, or at least a part of the components in the catalyst composition may form complex oxide. In the present invention, it is considered that an addition of diamond thereto further increases the catalyst activity.

The diamond is preferably located in the vicinity of a surface layer of the catalyst. A diamond content based on a weight of the ammoxidation catalyst is 0.1 % by weight or more, preferably 0.5 % by weight or more, further preferably 2 % by weight or more and particularly preferably 4 % by weight or more. The inventors have confirmed that the diamond itself does not have a strong oxidizing ability and that no problems are brought about on the catalyst performance if an addition amount of the diamond is increased more than necessary, but in that case, there is a disadvantage that the catalyst production cost is increased. Considering the above matter, the diamond content based on a weight of the ammoxidation catalyst is 70 % by weight or less, preferably 50 % by weight or less, further preferably 30 % by weight or less and particularly preferably 10 % by weight or less.

In catalysts for ammoxidation reaction, an addition of various components is a usual means for enhancing the reaction selectivity, and therefore catalysts having a higher selectivity tend to have larger number of components. When the added components are increased, anticipated are the troubles that an operation for preparing raw materials for the many components and a pre-treating operation for the catalyst are cumbersome and complicated and that a catalyst composition range and a reaction condition range for providing an optimum reaction result are narrowed. In a case of diamond, however, the physical and chemical stabilities thereof are very high, and a change in quality caused by reaction with other components and conversion to a different crystal phase are not easily brought about under usual catalyst preparing conditions, so that the above concerns are small. Diamond has little limitation regarding treating conditions on or after adding it and regarding storing conditions, and can be easily used as an addition component having a broad application range. Thus, no problems are involved in using publicly known catalyst producing processes as a process for producing the catalyst of the present invention.

As described above, a process for producing the catalyst containing the diamond is not specifically restricted. However, according to investigations of the present inventors, if the diamond and the oxides are more sufficiently dispersed and mixed in the catalyst, the higher catalyst activity and the higher reaction selectivity tend to be obtained, and therefore a production process which makes it possible to sufficiently disperse and mix them is particularly preferred.

A sufficiently dispersed and mixed state can be readily reached by carrying out an operation of adding fine powdery diamond to the raw material substances of the oxide components and then sufficiently dispersing or mixing them, as a part of the preparation step. The examples of a catalyst production process into which the above operation is introduced include (a) a process which includes adding fine powdery diamond to a homogeneous aqueous solution containing water-soluble raw materials of the respective oxide components, sufficiently stirring the resultant mixture to turn it into a suspension state, forming a precursor substance by evaporation of the water solvent or by co-precipitation using a suitable precipitating agent, and converting the precursor substance into an oxide mixture containing the diamond by an operation such as burning, and (b) a process which includes adding a part of oxide components and fine powdery diamond to a homogeneous aqueous solution containing water-soluble raw materials of a part of the oxide components, sufficiently stirring the resultant mixture to turn it into a suspension state, forming a precursor substance by evaporation of the water solvent or by co-precipitation using a suitable precipitating agent, and converting the precursor substance into an oxide mixture containing the diamond by an operation such as burning.

The ammoxidation catalyst employed in the present invention can be used as a catalyst in a reaction, without using a support, by processing itself into a granular substance or a molded matter. Publicly known methods can be used for processing it into a granular substance or a molded matter. The examples of the production process for the catalyst include (c) a process which includes adding fine powdery diamond to a homogeneous aqueous solution containing water-soluble raw materials of the respective oxide components, sufficiently stirring the resultant mixture to turn it into a suspension state, spraying the mixture in a suspension state to form a granular precursor, and converting the precursor into a granular catalyst of an oxide mixture containing the diamond by burning and the like, (d) a process which includes adding fine powdery diamond to a homogeneous aqueous solution containing water-soluble raw materials of the respective oxide components, sufficiently stirring the resultant mixture to turn it into a suspension state, forming a precursor substance by evaporation of the water solvent or co-precipitation using a suitable precipitating agent, molding the precursor substance by pelletizing, extruding, granulating and the like, and converting the molded precursor substance into a molded catalyst of an oxide mixture containing the diamond by burning and the like, (e) a process which includes forming a precursor substance by evaporation of a water solvent from a homogeneous aqueous solution containing water-soluble raw materials of a part of oxide components or by co-precipitation of the homogeneous aqueous solution by using a suitable precipitating agent, adding a part of the oxide components and fine powdery diamond to the precursor substance, sufficiently kneading the mixture, molding the mixture by pelletizing, extruding, granulating and the like, and converting the molded mixture into a molded catalyst of an oxide mixture containing the diamond by burning and the like and (f) a process which includes adding fine powdery diamond to a homogeneous aqueous solution containing water-soluble raw materials of the respective oxide components, sufficiently stirring the mixture to turn it into a suspension state, forming a precursor substance by evaporation of the water solvent or by co-precipitation using a suitable precipitating agent, converting the precursor substance into an oxide mixture containing the diamond by burning and the like, and turning the mixture into a molded catalyst by pelletizing, extruding, granulating and the like.

In the present invention, regarding a form and a particle size distribution of the granular substance and a form and a dimension of the molded matter, the ones which are the same as those having so far been publicly known and which are suited to the reactions can be employed. In a case of the molded matter, a suitable form thereof is spherical, cylindrical, ring-like and the like. In a case of the molded matter by pelletizing, a molding auxiliary agent (binder) such as graphite and ethyl cellulose is preferably added in compressing and molding the powder.

The ammoxidation catalyst employed in the present invention can be used in the form of a supported catalyst, which is a catalyst supported on a support inert to the reaction. Materials for the support are not specifically restricted, and publicly known ones can be used. Preferred examples of the support material include silicon carbide, alumina, silica, zirconia, steatite, cordierite, mullite, ceramics and porcelains. In the present invention, a molded support containing at least one selected from the above support materials is preferably used. A form of the support is not specifically restricted as well, and supports having publicly known forms such as spherical, cylindrical and ring-like ones are suitably used. Further, the physical properties of the support such as a dimension, a porosity and a BET specific surface area are not restricted as well, and supports which are the same as publicly known ones and which are suited to the reactions can be employed.

A method for supporting the catalyst employed in the present invention on the support is not specifically restricted, and publicly known methods can be used. Preferred is a supporting method in which consideration is given for the diamond and the oxides to be more sufficiently dispersed and mixed in the catalyst, as described above. From such a viewpoint, preferred examples of the supporting method are (g) a method which includes adding fine powdery diamond to a homogeneous aqueous solution containing water-soluble raw materials of the respective oxide components, sufficiently stirring the resultant mixture to turn it into a suspension state, spraying the suspension on a support so that the suspension can be supported on the support, and converting it into a supported catalyst of an oxide mixture containing the diamond by burning and the like and (h) a method which includes adding a part of oxide components and fine powdery diamond to a homogeneous aqueous solution containing water-soluble raw materials of a part of the oxide components, sufficiently stirring the resultant mixture to turn it into a suspension state, spraying the suspension on a support so that the suspension ban be supported on the support, and converting it into a supported catalyst of an oxide mixture containing the diamond by burning and the like.

In the supported catalyst described above, an amount of the supported ammoxidation catalyst based on a total weight of the ammoxidation catalyst and the support is preferably 0.1 to 20 % by weight, more preferably 0.5 to 15 % by weight and further preferably 1 to 10 % by weight. An amount of the supported catalyst of 20 % by weight or less is preferred, because , in that case, the supported components are less liable to be peeled off, and an amount of the supported catalyst of 0.1 % by weight or more is preferred, because, in that case, the satisfactory reaction activity is obtained.

### [Production process for nitrile compound]

A production process for a nitrile compound of the present invention includes an ammoxidation reaction step in which an organic compound is reacted in a gas phase with a mixed gas containing ammonia and oxygen under the presence of the ammoxidation catalyst described above. The nitrile compound obtained by the above process may be either a mononitrile compound or a polynitrile compound.

### (Organic compound)

The organic compound used in the production process for a nitrile compound of the present invention has at least one organic substituent which provides a nitrile group by ammoxidation reaction in a molecule. Examples of the above organic substituent include methyl, ethyl, propyl, formyl, acetyl, hydroxymethyl and methoxycarbonyl. Among them, methyl, ethyl, propyl, acetyl and formyl are preferred, and methyl is more preferred. When the organic compound has plural organic substituents in a molecule, they may be the same or different. The organic compound having the above organic substituent may be any of saturated aliphatic hydrocarbons, unsaturated aliphatic hydrocarbons, aromatic hydrocarbons, heterocyclic compounds, alcohol compounds, aldehyde compounds and ketone compounds. Also, intermediates produced during the course of ammoxidation reaction of the above compounds, the carbon atoms of which are partially converted through the ammoxidation reaction to nitrile groups, can be used without being specifically restricted, as long as they can be evaporated to be subjected to ammoxidation reaction on the catalyst. The organic compound can be used in a single kind selected from the above or in a mixture of two or more kinds thereof. When different two or more kinds of the organic compounds are used, a mixing proportion thereof is not restricted.

The saturated aliphatic hydrocarbons are not specifically restricted as long as they are liner, branched or cyclic alkanes. Examples thereof include methane, ethane, propane, n-butane, isobutane, 2-methylbutane, 2,2-dimethylbutane, n-hexane, 2,2,4-trimethylpentane, cyclopentane, cyclohexane and decalin.

The unsaturated aliphatic hydrocarbons are not specifically restricted as long as they are unsaturated compounds in which at least one of C-C single bonds in linear, branched or cyclic alkanes is replaced with a C-C double bond. When they are the compounds replaced with two or more double bonds, the double bonds may be or may not be conjugated. Also, when geometric isomers are present, the compounds may be either individual isomers or mixtures of the isomers. Examples thereof include ethylene, propylene, 1-butene, 2-butene, isobutene, 1,3-butadiene, 2-methyl-1,3-butadiene, 1-hexene, 1,5-hexadiene, cyclopentene, cyclopentadiene and cyclohexene.

The aromatic hydrocarbons are not specifically restricted as long as they are compounds having at least one aromatic ring. A linear, branched or cyclic alkyl group and an alkenyl group may be bonded to the aromatic ring. When the aromatic hydrocarbons have plural aromatic rings, the rings themselves may be condensed, directly bonded or bonded via at least one carbon chain outside the ring. Examples thereof include benzene, toluene, ethylbenzene, n-propylbenzene, i-propylbenzene, n-butylbenzene, o-xylene, m-xylene, p-xylene, 1,2,4-trimethylbenzene and 1,2,4,5-tetramethylbenzene.

The heterocyclic compounds are not specifically restricted as long as they are compounds having at least one heterocyclic ring. A linear, branched or cyclic alkyl group, an alkenyl group and an aryl group may be bonded to the heterocyclic ring. When the heterocyclic compounds have plural heterocyclic rings, the rings themselves may be condensed, directly bonded or bonded via at least one carbon chain outside the ring. Examples thereof include 2-methylpyridine, 2-methyl-5-ethylpyridine, 3-methylpyridine and 4-methylpyridine.

The alcohol compounds are not specifically restricted as long as they are compounds in which at least one of C-H bonds in the compounds selected from the saturated aliphatic hydrocarbons, the unsaturated aliphatic hydrocarbons, the aromatic hydrocarbons and the heterocyclic compounds each described above is replaced with a C-OH bond (hydroxyl group). Examples thereof include methanol, ethanol, 1-propanol, isopropanol, 1-butanol and isobutanol.

The aldehyde compounds are not specifically restricted as long as they are compounds in which at least one of end methyl (CH₃) groups in the compounds selected from the saturated aliphatic hydrocarbons, the unsaturated aliphatic hydrocarbons, the aromatic hydrocarbons and the heterocyclic compounds each described above is replaced with a formyl (CHO) group. Examples thereof include formaldehyde, acetaldehyde, propionaldehyde, n-butylaldehyde, caproaldehyde, acrolein, methacrolein, crotonaldehyde, succinaldehyde, malealdehyde, glutaraldehyde, benzaldehyde, o-tolualdehyde, m-tolualdehyde, p-tolualdehyde and nicotinealdehyde.

The ketone compounds are not specifically restricted as long as they are compounds in which at least one of methylene (CH₂) groups in the compounds selected from the saturated aliphatic hydrocarbons, the unsaturated aliphatic hydrocarbons, the aromatic hydrocarbons and the heterocyclic compounds each described above is replaced with a carbonyl (C=O) group. The examples thereof include acetone, 2-butanone, 2-pentanone, pinacolone, 2-hexanone, 2,3-butanedione, 2,4-pentanedione, cyclopentanone, cyclohexanone and methyl vinyl ketone.

The production process for a nitrile compound of the present invention is suitably carried out when the organic compound is a carbocyclic compound such as aromatic compounds and alicyclic compounds or a heterocyclic compound. When the organic compound is an aromatic compound having the organic substituent, the organic substituent can be converted through ammoxidation reaction into a nitrile group while maintaining the aromatic ring by controlling an oxidation power of the catalyst, and thus an aromatic nitrile compound can be produced. The aromatic nitrile compound produced in this manner is useful as various industrial raw materials and therefore shall be described below in detail.

A raw material compound which provides the aromatic nitrile compound according to the present invention is an aromatic compound having 7 to 20 carbon atoms which has at least one aromatic ring in a molecule and which has at least one organic substituent bonded to the aromatic ring, and examples of the organic substituent bonded to the aromatic ring include methyl, ethyl, propyl (n-propyl and i-propyl), formyl, acetyl, hydroxymethyl and methoxycarbonyl. Among the above organic substituents, methyl, ethyl, propyl, acetyl and formyl are preferred, and methyl is more preferred. Further, the organic substituent may be bonded to a different site on the same aromatic ring to form a cyclic structure. When the aromatic compound has plural aromatic rings in a molecule, the rings themselves may be condensed, directly bonded or bonded via at least one carbon chain outside the ring. When the aromatic compound has plural organic substituents in a molecule, the organic substituents may be the same or different, and a positional relation thereof is not restricted. A location of the organic substituents when the aromatic compound has plural aromatic rings is not restricted as well. Also, if these compounds contain a halogen group, a hydroxy group, an alkoxyl group, an amino group, a nitrile group or the like, they can be used as well.

Further, intermediates produced during the course of converting all of the organic substituents through ammoxidation reaction to nitrile groups can also be used as the raw material compound. Examples thereof include aromatic compounds in which at least one organic substituent has been converted through ammoxidation reaction to a nitrile group and which still have organic substituents capable of being converted through ammoxidation reaction to nitrile groups in a molecule.

The above aromatic compounds can be used as the raw material compounds in a single kind selected from them or in a mixture of two or more kinds thereof without being specifically restricted, as long as they can be evaporated to be subjected to ammoxidation reaction on the catalyst. When different two or more kinds of the aromatic compounds are used, a mixing proportion thereof is not restricted. Examples of the aromatic compound having the organic substituent include toluene, ethylbenzene, n-propylbenzene, i-propylbenzene, n-butylbenzene, o-xylene, m-xylene, p-xylene, 1,2,4-trimethylbenzene, 1,2,4,5-tetramethylbenzene, 1-methylnaphthalene, 2-methylnaphthalene, 1,5-dimethylnaphthalene, 1,4-dimethylnaphthalene, 2,3-dimethylnaphthalene, 2,6-dimethylnaphthalene, 2,6-diethylnaphthalene, 2,7-dimethylnaphthalene, biphenyl, 4-methylbiphenyl, 4,4'-dimethylbiphenyl and 4,4'-diethylbiphenyl. Among them, preferred are toluene, ethylbenzene, n-propylbenzene, i-propylbenzene, n-butylbenzene, o-xylene, m-xylene, p-xylene, 1,2,4-trimethylbenzene and 1,2,4,5-tetramethylbenzene, and o-xylene, m-xylene and p-xylene are more preferred.

For example, if o-xylene, m-xylene and p-xylene which are three isomers of xylene are used as the aromatic compounds, aromatic nitriles such as benzonitrile, orthophthalonitrile, isophthalonitrile and terephthalonitrile can be produced.

### (Ammonia)

The raw material ammonia used in the present invention may be an industrial grade. A use amount of the ammonia falls in a range of preferably 1 to 20 times mole, more preferably 3 to 15 times mole based on one organic substituent contained in 1 mole of the raw material organic compound. The targeted products can be obtained at a good yield by controlling the use amount to the above ranges. In the process of the present invention, unreacted ammonia contained in the reaction gas can be reused by recovering it and turning it to the reaction system. Various methods for recovering the unreacted ammonia from the reaction gas can be considered, but it is industrially advantageous that unreacted ammonia is absorbed in water and that the solution is then subjected to distilling operation to separate ammonia from other by-products. A moisture amount of the ammonia recovered above is varied depending on the operation conditions of the distillation, but a moisture is usually contained in an amount of 5 to 20 % by volume.

### (Oxygen)

Oxygen used in the present invention is not specifically restricted as long as it is supplied in the form of an oxygen-containing gas, which contains molecular oxygen, but air is usually used. In another embodiment, air, an oxygen gas or mixture of them after dilution can be used, wherein the dilution is performed by mixing them with inert gases such as nitrogen, carbon dioxide, helium and argon or exhaust gases in an optional ratio. A use amount of the oxygen falls in a range of preferably 1.5 times mole or more, more preferably 2 to 50 times mole in terms of O₂ based on one organic substituent contained in 1 mole of the raw material organic compound. The targeted products can be obtained at a good yield by controlling the use amount to the above ranges.

The reaction form of the ammoxidation reaction in the present invention is not specifically restricted, but it is usually a gas phase flow fixed bed form. A filling layer catalyst reactor in which the catalyst particles are filled and in which the gases of the raw materials are continuously reacted is usually used as the reaction apparatus. In a case of the fixed bed form, a molded catalyst which is composed of a non-supported catalyst or a molded catalyst which is supported on a support is usually used, and catalyst physical properties such as a catalyst form and a dimension and reaction conditions suitable for carrying out the ammoxidation reaction under a fixed state are employed. In the case of the fixed bed form, plural kinds of catalysts different in catalyst composition and catalyst component amounts are usually used by filling them into a reaction tube in a multilayer. In the above case, a catalyst containing the catalyst of the present invention can be used for at least one optional filling layer of or all filling layers of the multi filling layers.

A heat transfer system of the filling layer catalyst reactor is classified roughly into an adiabatic system and a heat exchange system. Since the reaction in the present invention is an exothermic reaction, the heat exchange system is preferably used. In particular, preferred is a single-tubular or multi-tubular heat exchange system, in which a catalyst is filled in a reaction tube and a heat transfer medium is passed outside of the tube.

Materials for the reactor depend on the kind of the raw materials and the reaction conditions, but, in general, a stainless steel and a carbon steel are preferred.

A diameter of the reaction tube is preferably 1 to 5 cm, and a length thereof is preferably 10 cm to 7 m.

In respect to the reaction temperature, the reaction can be carried out in a wide range of 250 to 550°C in terms of a heat transfer medium temperature, and it is preferably 330 to 470°C. The reaction temperature lower than the above range is not preferred, because, in that case, the satisfactory reaction rate is not obtained and a conversion ratio of the raw material compound is small. On the other hand, the reaction temperature higher than the above range is not preferred, because, in that case, the complete combustion reaction is predominant at high temperature and production of carbon dioxide is increased, so that the nitrile compound which is an ammoxidation reaction product is reduced in a yield.

The heat transfer medium is particularly preferably a molten salt, because the reaction is carried out at high temperature. The reaction temperature showing the highest yield is varied according to the kind of the raw material, the raw material concentration, the contact time and a burning temperature of the catalyst, and therefore it is preferable to select a reaction temperature suitably within the above ranges according to the conditions. A contact time of the reaction gas with the catalyst can be set usually in a considerably broad range, and it is preferably 0.5 to 30 seconds. The gas space velocity (SV) is preferably 500 to 10000 hr⁻¹, more preferably 1000 to 8000 hr⁻¹.

The reaction of the present invention is carried out usually at atmospheric pressure, but it can be carried out as well under applied pressure or reduced pressure. The reaction product is collected by using an optional suitable method, for example, a method in which it is collected by cooling down to temperature enough for the product to be deposited, and a method in which it is collected by washing the reaction product gas with water or other suitable solvents. The reaction product collected is refined by a publicly known method such as distillation, whereby the nitrile compound which is the targeted substance is obtained.

### Examples

The present invention shall be explained below in further details with reference to examples and comparative examples, but the present invention is not restricted to these examples as long as it does not deviate from a scope thereof.

### <Example 1>

Chromic anhydride CrO₃ (manufactured by Wako Pure Chemical Industries, Ltd.) 19.6 g was dissolved in 20 mL of purified water, and 60 mL of purified water was added to 75.3 g of oxalic acid (manufactured by Wako Pure Chemical Industries, Ltd.) and heated at 50 to 60°C. The chromic acid aqueous solution described above was gradually added to the above aqueous solution while stirring to prepare a chromium oxalate aqueous solution. On the other hand, 44.4 g of oxalic acid was dissolved in 40 mL of purified water and heated at 80 to 90°C, and then 17.8 g of vanadium pentaoxide V₂O₅ (manufactured by Wako Pure Chemical Industries, Ltd.) was gradually added thereto while stirring well to prepare a vanadyl oxalate aqueous solution. Next, the chromium oxalate aqueous solution prepared was dropwise added to the vanadyl oxalate aqueous solution prepared at 70 to 90°C to mix them. Boric acid 1.21 g was added to the above mixed aqueous solution at 70 to 90°C to mix them. The catalyst solution thus prepared was heated at 85 to 95°C to become ripened, and then the solution was concentrated at 100 to 110°C. Anatase-type titanium oxide 133.3 g was added to the concentrated prepared liquid, and kneaded by means of a kneader at 70°C with evaporating the moisture until the mixture became homogeneous. Then, the cake thus obtained was dried on the condition of 110°C in a drying equipment.

Next, the dried product was pre-baked on the condition of 500°C for 2 hours in a baking furnace and then crushed with a crushing equipment.

Graphite 4 % by weight (based on a weight of the crushed powder) and powdery natural diamond (product passing through a sieve having an aperture of 1 µm) 5 % by weight (based on a weight of the crushed powder) were added to the crushed powder and mixed. Next, the above raw material powder was pelletized and molded by means of a pellet molding equipment so that it was turned into a ring-like form having an outer diameter of 6 mm, an inner diameter of 3 mm and a height of 6 mm. The molded product was baked at 550°C for 15 hours in a baking furnace. A composition of this catalyst is shown in Table 1. The added graphite was used as a lubricant for improving lubrication in carrying out the pelletizing and molding. The graphite was burned up in the baking step after the molding, and an amount of the graphite remaining in the catalyst was reduced to an ignorable amount.

The catalyst 1.5 kg was produced by repeating the catalyst preparation in accordance with the method described above, and it was filled into a carbon steel reaction tube having an inner diameter of 30 mm. The reaction tube was set in a molten salt bath maintained at a prescribed temperature (380°C), and pipes in an inlet side and an outlet side of the reaction tube were kept heated by heaters. The raw material was gasified in an evaporator maintained at 130°C, and a gas composed of 1.0 % by volume of meta-xylene, 8.0 % by volume of ammonia, 5.0 % by volume of oxygen and 86.0 % by volume of nitrogen was introduced into the reaction tube and subjected to catalytic reaction on the condition of a gas space velocity (SV) of 2400 Hr⁻¹. The reaction product obtained after 24 hours passed since the reaction was started was analyzed by a gas chromatography. As a result thereof, a yield of isophthalonitrile based on meta-xylene was 91 %.

### <Example 2> (Changed from natural diamond to artificial diamond)

A catalyst was prepared by the same operation as in Example 1, except that the kind of the diamond was changed to powdery artificial diamond (product passing through a sieve having an aperture of 1 µm). Meta-xylene was subjected to ammoxidation reaction by the same method as in Example 1, and the results thereof are shown in Table 1.

### <Example 3> (Changed in an addition amount of natural diamond)

A catalyst was prepared by the same operation as in Example 1, except that an addition amount of the diamond was reduced to 1.0 % by weight (based on a weight of the crushed powder). Meta-xylene was subjected to ammoxidation reaction by the same method as in Example 1, and the results thereof are shown in Table 1.

### <Example 4> (Changed in an addition amount of artificial diamond)

A catalyst was prepared by the same operation as in Example 2, except that an addition amount of the diamond was reduced to 1.0 % by weight (based on a weight of the crushed powder). Meta-xylene was subjected to ammoxidation reaction by the same method as in Example 1, and the results thereof are shown in Table 1.

### <Comparative Example 1> (No diamond added)

A catalyst was prepared by the same operation as in Example 1, except that the preparation was made without adding diamond. Meta-xylene was subjected to ammoxidation reaction by the same method as in Example 1, and the results thereof are shown in Table 1.

### <Example 5> (Changed from meta-xylene to para-xylene)

Para-xylene was subjected to ammoxidation reaction by the same method as in Example 1 with setting the molten salt bath to 370°C, in which a catalyst prepared by the same operation as in Example 1 was used. As a result thereof, a yield of terephthalonitrile was 92 %. The results thereof are shown in Table 1.

### <Comparative Example 2> (Para-xylene used; no diamond added)

Para-xylene was subjected to ammoxidation reaction by the same method as in Example 1 with setting the molten salt bath to 370°C, in which a catalyst prepared by the same operation as in Example 5 except that the preparation was made without adding diamond was used. As a result thereof, a yield of terephthalonitrile was 87 %. The results thereof are shown in Table 1.

### <Example 6> (No Cr added)

Oxalic acid 44.4 g was dissolved in 40 mL of purified water and heated at 80 to 90°C, and then 17.8 g of vanadium pentaoxide V₂O₅ (manufactured by Wako Pure Chemical Industries, Ltd.) was gradually added thereto while stirring well to prepare a vanadyl oxalate aqueous solution. Boric acid 1.21 g was added to the above aqueous solution at 70 to 90°C to mix them. The catalyst solution thus prepared was heated at 85 to 95°C to become ripened, and then the solution was concentrated at 100 to 110°C. Anatase type titanium oxide 133.3 g was added to the concentrated prepared liquid, and kneaded by means of a kneader at 70°C with evaporating the moisture until the mixture became homogeneous. Then, the cake thus obtained was dried on the condition of 110°C in a drying equipment.

Next, the dried product was pre-baked on the condition of 500°C for 2 hours in a baking furnace and then crushed with a crushing equipment.

With regard to the subsequent procedures for preparing a catalyst, the same operation as in Example 1 was made. Meta-xylene was subjected to ammoxidation reaction by the same method as in Example 1 with setting the molten salt bath to 360°C, and the results thereof are shown in Table 1.

### <Comparative Example 3> (No Cr added; no diamond added)

A catalyst was prepared by the same operation as in Example 6, except that the preparation was made without diamond. Meta-xylene was subjected to ammoxidation reaction by the same method as in Example 1 with setting the molten salt bath to 360°C, and the results thereof are shown in Table 1.

### <Example 7> (No Cr added; Mo added)

Oxalic acid 44.4 g was dissolved in 40 mL of purified water and heated at 80 to 90°C, and then 17.8 g of vanadium pentaoxide V₂O₅ (manufactured by Wako Pure Chemical Industries, Ltd.) was gradually added thereto while stirring well to prepare a vanadyl oxalate aqueous solution. Next, 3.5 g of molybdenum ammonium-tetrahydrate (manufactured by Wako Pure Chemical Industries, Ltd.) was added thereto at 70 to 90°C and mixed. Boric acid 1.21 g was added to the above mixed aqueous solution at 70 to 90°C to mix them. The catalyst solution thus prepared was heated at 85 to 95°C to become ripened, and then the solution was concentrated at 100 to 110°C. Anatase type titanium oxide 133.3 g was added to the concentrated prepared liquid, and kneaded by means of a kneader at 70°C with evaporating the moisture until the mixture became homogeneous. Then, the cake thus obtained was dried on the condition of 110°C in a drying equipment.

Next, the dried product was pre-baked on the condition of 500°C for 2 hours in a baking furnace and then crushed with a crushing equipment.

With regard to the subsequent steps for preparing a catalyst, the same operation as in Example 1 was made. Meta-xylene was subjected to ammoxidation reaction by the same method as in Example 1, and the results thereof are shown in Table 1.

### <Comparative Example 4> (No Cr added; Mo added; no diamond added)

A catalyst was prepared in the same operation as in Example 7, except that the preparation was made without adding diamond. Meta-xylene was subjected to ammoxidation reaction by the same method as in Example 1, and the results thereof are shown in Table 1.

### <Example 8> (No Cr added; P added)

Oxalic acid 44.4 g was dissolved in 40 mL of purified water and heated at 80 to 90°C, and then 17.8 g of vanadium pentaoxide V₂O₅ (manufactured by Wako Pure Chemical Industries, Ltd.) was gradually added thereto while stirring well to prepare a vanadyl oxalate aqueous solution. Next, 3.5 g of ammonium dihydrogen phosphate (manufactured by Wako Pure Chemical Industries, Ltd.) was added thereto at 70 to 90°C and mixed. Boric acid 1.21 g was added to the above mixed aqueous solution at 70 to 90°C to mix them. The catalyst solution thus prepared was heated at 85 to 95°C to become ripened, and then the solution was concentrated at 100 to 110°C. Anatase type titanium oxide 133.3 g was added to the concentrated prepared liquid, and kneaded by means of a kneader at 70°C with evaporatin the moisture until the mixture became homogeneous. Then, the cake thus obtained was dried on the condition of 110°C in a drying equipment.

Next, the dried product was pre-baked on the condition of 500°C for 2 hours in a baking furnace and then crushed with a crushing equipment.

With regard to the subsequent steps for preparing a catalyst, the same operation as in Example 1 was made. Meta-xylene was subjected to ammoxidation reaction by the same method as in Example 1, and the results thereof are shown in Table 1.

### <Comparative Example 5> (No Cr added; P added; no diamond added)

A catalyst was prepared by the same operation as in Example 8, except that the preparation was made without adding diamond. Meta-xylene was subjected to ammoxidation reaction by the same method as in Example 1, and the results thereof are shown in Table 1.

### <Example 9> (No B added)

A catalyst was prepared by the same operation as in Example 1, except that boric acid was not added. Meta-xylene was subjected to ammoxidation reaction by the same method as in Example 1, and the results thereof are shown in Table 1.

### <Comparative Example 6> (No B added; no diamond added)

A catalyst was prepared in the same operation as in Example 9, except that the preparation was made without adding diamond. Meta-xylene was subjected to ammoxidation reaction by the same method as in Example 1, and a result thereof is shown in Table 1.

### <Example 10> (No Cr added; No B added)

Oxalic acid 44.4 g was dissolved in 40 mL of purified water and heated at 80 to 90°C, and then 17.8 g of vanadium pentaoxide V₂O₅ (manufactured by Wako Pure Chemical Industries, Ltd.) was gradually added thereto while stirring well to prepare a vanadyl oxalate aqueous solution. The above catalyst solution was heated at 85 to 95°C to become ripened, and then the solution was concentrated at 100 to 110°C. Anatase type titanium oxide 133.3 g was added to the concentrated prepared liquid, and kneaded by means of a kneader at 70°C with evaporating the moisture until the mixture became homogeneous. Then, the cake thus obtained was dried on the condition of 110°C in a drying equipment.

Next, the dried product was pre-baked on the condition of 500°C for 2 hours in a baking furnace and then crushed with a crushing equipment.

With regard to the subsequent steps for preparing a catalyst, the same operation as in Example 1 was made. Meta-xylene was subjected to ammoxidation reaction by the same method as in Example 1 with setting the molten salt bath to 355°C, and the results thereof are shown in Table 1.

### <Comparative Example 7> (No Cr added; no B added; no diamond added)

A catalyst was prepared by the same operation as in Example 10, except that the preparation was made without adding diamond. Meta-xylene was subjected to ammoxidation reaction by the same method as in Example 1 with setting the molten salt bath to 355°C , and the results thereof are shown in Table 1.

### <Example 11> (Supported catalyst)

Chromic anhydride CrO₃ 3.27 g was dissolved in 20 mL of purified water, and 10 mL of purified water was added to 12.55 g of oxalic acid and heated at 50 to 60°C. The chromic acid aqueous solution described above was gradually added to the above aqueous solution while stirring to prepare a chromium oxalate aqueous solution. On the other hand, 7.4 g of oxalic acid was dissolved in 7 mL of purified water and heated at 80 to 90°C, and then 2.97 g of vanadium pentaoxide V₂O₅ was gradually added thereto while stirring well to prepare a vanadyl oxalate aqueous solution. Next, the chromium oxalate aqueous solution prepared was dropwise added to the vanadyl oxalate aqueous solution prepared at 70 to 90°C to mix them. Boric acid 0.2 g was added to the above mixed aqueous solution at 70 to 90°C to mix them. Further, 9.56 g of ammonium titanyl oxalate [(NH₄)₂TiO(C₂H₄)₂] (manufactured by Soekawa Chemical Co., Ltd.) was dissolved in 40 mL of purified water heated at 50°C to prepare a raw material solution. This solution was added to the mixed solution prepared above, and 33.33 g of commercial powdery anatase type titanium dioxide (BET specific surface area: 28.0 m²/g) and 3.00 g of commercial powdery natural diamond (average particle diameter: 0.1 µm) were mixed well with the above solution to prepare a slurry solution.

With heating a silicon carbide support (ring-like molded product: outer diameter 6□ ×inner diameter 3□ × height 6 mm, BET specific surface area: 0.05 m²/g, pore volume: 0.24 ml/g and porosity: 44.3 %) at 150°C or higher, the slurry solution described above was sprayed on it to be supported on it, and subsequently the resultant product was subjected to air baking treatment at 550°C for 10 hours in an electric furnace to thereby produce a catalyst. An amount and an composition of the supported catalyst are shown in Table 1.

A tubular reactor (inner diameter: 18 mm, length: 500 mm) was charged with 17 g (including a weight of the support) of the above catalyst and dipped in a molten salt (co-molten substance of potassium nitrate and sodium nitrate) bath. A temperature of the molten salt bath was controlled to 390°C, and a gas composed of 1.0 % by volume of meta-xylene, 8.0 % by volume of ammonia, 5.0 % by volume of oxygen and 86.0 % by volume of nitrogen was introduced into the reaction tube and subjected to catalytic reaction on the condition of a gas space velocity (SV) of 2400 Hr⁻¹. The reaction product obtained after 24 hours passed since the reaction was started was analyzed by a gas chromatography, and the results thereof are shown in Table 2.

### <Example 12> (Supported catalyst: changed from natural diamond to artificial diamond)

A catalyst was prepared by the same operation as in Example 11, except that the kind of the diamond was changed to powdery artificial diamond (average particle diameter: 0.1 µm). Meta-xylene was subjected to ammoxidation reaction by the same method as in Example 11, and the results thereof are shown in Table 2.

### <Comparative Example 8> (Supported catalyst: no diamond added)

A catalyst was prepared by the same operation as in Example 11, except that the preparation was made without adding diamond. Meta-xylene was subjected to ammoxidation reaction by the same method as in Example 11, and the results thereof are shown in Table 2.

**Table 1 Unsupported catalysts**

| | Catalyst composition (wt %) | | | | | | | Diamond kind | Reaction | Molten salt bath temperature (°C) | Phthalonitrile yield (%) |
|---|---|---|---|---|---|---|---|---|---|---|---|
| | V₂O₅ | Cr₂O₃ | TiO₂ | MnO₃ | P₂O₅ | B₂O₃ | Diamond | | | | |
| Example 1 | 10.2 | 8.5 | 76.2 | | | 0.4 | 4.8 | Natural | MX→IPN | 380 | 91 |
| Example 2 | 10.2 | 8.5 | 76.2 | | | 0.4 | 4.8 | Artificial | MX→IPN | 380 | 89 |
| Example 3 | 10.6 | 8.8 | 79.2 | | | 0.4 | 1.0 | Natural | MX→IPN | 380 | 89 |
| Example 4 | 10.6 | 8.8 | 79.2 | | | 0.4 | 1.0 | Artificial | MX→IPN | 380 | 87 |
| Comparative Example 1 | 10.7 | 8.9 | 80.0 | | | 0.4 | - | - | MX→IPN | 380 | 86 |
| Example 5 | 10.2 | 8.5 | 76.2 | | | 0.4 | 4.8 | Natural | PX→TPN | 370 | 92 |
| Comparative Example 2 | 10.7 | 8.9 | 80.0 | | | 0.4 | - | - | PX→TPN | 370 | 87 |
| Example 6 | 11.2 | | 83.6 | | | 0.4 | 4.8 | Natural | MX→IPN | 360 | 82 |
| Comparative Example 3 | 11.8 | | 87.8 | | | 0.4 | - | - | MX→IPN | 360 | 78 |
| Example 7 | 11.0 | | 82.9 | 0.9 | | 0.4 | 4.8 | Natural | MX→IPN | 380 | 87 |
| Comparative Example 4 | 11.7 | | 87.0 | 0.9 | | 0.4 | - | - | MX→IPN | 380 | 82 |
| Example 8 | 11.0 | | 82.9 | | 0.9 | 0.4 | 4.8 | Natural | MX→IPN | 380 | 85 |
| Comparative Example 5 | 11.7 | | 87.0 | | 0.9 | 0.4 | - | - | MX→IPN | 380 | 80 |
| Example 9 | 10.2 | 8.5 | 76.5 | | | | 4.8 | Natural | MX→IPN | 380 | 79 |
| Comparative Example 6 | 10.7 | 9.0 | 80.3 | | | | - | - | MX→IPN | 380 | 75 |
| Example 10 | 11.4 | | 83.8 | | | | 4.8 | Natural | MX→IPN | 355 | 77 |
| Comparative Example 7 | 12.0 | | 88.0 | | | | - | - | MX→IPN | 355 | 72 |

| | | | | | | | | | | | |
|---|---|---|---|---|---|---|---|---|---|---|---|
| MX: meta-xylene, IPN: isophthalonitrile PX: para-xylene, TPN: terephthalonitrile | | | | | | | | | | | |

**Table 2 Supported catalysts**

| | Catalyst composition (wt %) | | | | | Amount of supported catalyst (wt %) | Diamond kind | Reaction | Molten salt bath temperature (°C) | Phthalonitrile yield (%) |
|---|---|---|---|---|---|---|---|---|---|---|
| | V₂O₅ | Cr₂O₃ | TiO₂ | B₂O₃ | Diamond | | | | | |
| Example 11 | 6.6 | 5.6 | 80.8 | 0.2 | 6.8 | 4.3 | Natural | MX→IPN | 390 | 90 |
| Example 12 | 6.6 | 5.6 | 80.8 | 0.2 | 6.8 | 4.3 | Artificial | MX→IPN | 390 | 88 |
| Comparative Example 8 | 7.1 | 6.0 | 86.6 | 0.3 | - | 4.0 | - | MX→IPN | 390 | 86 |

| | | | | | | | | | | |
|---|---|---|---|---|---|---|---|---|---|---|
| MX: meta-xylene, IPN: isophthalonitrile Amount of supported catalyst = (weight of catalyst)/(weight of catalyst + weight of support SiC) | | | | | | | | | | |

It can be found from the results of Example 1 to 4 and Comparative Example 1 shown in Table 1 described above that the yields of nitrile compounds were improved by adding the diamond in the catalysts. The satisfactory effects were obtained even when the artificial diamond was used, and the higher effects were obtained when the natural diamond was used. Further, when the addition amount was suitably increased, the higher effects were obtained. It seems from the results shown in the above tables as if the range of the improvement in the yield were small, but as is well known for persons ordinarily skilled in the art, an economical improving effect by an improvement by 1 % in yield of the above industrial catalyst is sufficiently large.

It can be found from the results obtained in Example 5 and Comparative Example 2 that the yield of nitrile compound was improved by addition of the diamond even when the reactant was changed.

As can be seen from the results obtained in Examples 6 to 10 and Comparative Examples 3 to 7, an addition effect of the diamond was exerted consistently even when the catalyst composition was changed.

Further, the examples of the supported catalysts prepared by conventional methods are shown in Table 2, and an addition effect of the diamond is sufficiently exerted likewise.

### Industrial Applicability

The yield and selectivity in the ammoxidation reaction can be enhanced by use of the ammoxidation catalyst employed in the present invention. In production of a nitrile compound, the yield of the nitrile compound can be enhanced by use of the ammoxidation catalysts described above.

## Claims

1. A process for producing a nitrile compound comprising an ammoxidation reaction step in which an organic compound is reacted in a gas phase with a mixed gas comprising ammonia and oxygen under the presence of an ammoxidation catalyst comprising vanadium oxide, titanium oxide and diamond, wherein the content of diamond is 0.1 to 70 % by weight based on the weight of the ammoxidation catalyst.

2. The process for producing a nitrile compound according to claim 1, wherein the organic compound is a carbocyclic compound or a heterocyclic compound.

3. The process for producing a nitrile compound according to claim 1, wherein the organic compound has at least one organic substituent selected from the group consisting of methyl, ethyl, propyl, formyl, acetyl, hydroxymethyl and methoxycarbonyl in a molecule.

4. The process for producing a nitrile compound according to claim 1, wherein the organic compound has at least one organic substituent selected from the group consisting of methyl, ethyl, propyl, acetyl and formyl in a molecule.

5. The process for producing a nitrile compound according to claim 1, wherein the organic compound is at least one selected from o-xylene, m-xylene and p-xylene.

6. The process for producing a nitrile compound according to claim 1, wherein the gas phase reaction in the ammoxidation reaction step is carried out by a fixed bed flow form.

7. The process for producing a nitrile compound according to any of claims 1 to 6, wherein the diamond comprised in the ammoxidation catalyst has a particle diameter of from more than 0.001 to 10 µm, measured by means of an existing laser dispersion type particle size measuring equipment.

8. The process for producing a nitrile compound according to any of claims 1 to 6, wherein the ammoxidation catalyst further comprises at least one oxide selected from the group consisting of chromium oxide, molybdenum oxide, tungsten oxide, zirconium oxide, iron oxide, cobalt oxide, nickel oxide, niobium oxide, boron oxide, aluminum oxide, germanium oxide, phosphorus oxide, antimony oxide and bismuth oxide.

9. The process for producing a nitrile compound according to any of claims 1 to 6, wherein the ammoxidation catalyst further comprises at least one oxide selected from the group consisting of chromium oxide, molybdenum oxide, iron oxide, boron oxide and phosphorus oxide.

10. The process for producing a nitrile compound according to any of claims 1 to 6, wherein the ammoxidation catalyst is supported on a support inert to the reaction.

11. The process for producing a nitrile compound according to claim 10, wherein the support is a molded support comprising at least one selected from the group consisting of silicon carbide, alumina, silica, zirconia, steatite, cordierite, mullite, ceramics and porcelains.

12. The process for producing a nitrile compound according to claim 10, wherein an amount of the supported ammoxidation catalyst is 0.1 to 20 % by weight based on a total weight of the ammoxidation catalyst and the support.

## Patentansprüche

1. Verfahren zur Herstellung einer Nitrilverbindung, umfassend einen Ammoxidierungs-Reaktionsschritt, in dem eine organische Verbindung in der Gasphase mit einem gemischten Gas, umfassend Ammoniak und Sauerstoff, in Gegenwart eines Ammoxidierungskatalysators, umfassend Vanadiumoxid, Titanoxid und Diamant, wobei der Gehalt an Diamant in bezug auf das Gewicht des Ammoxidierungskatalysators 0,1 bis 70 Gew.% beträgt, umgesetzt wird.

2. Verfahren zur Herstellung einer Nitrilverbindung gemäss Anspruch 1, wobei die organische Verbindung eine carbocyclische Verbindung oder eine heterocyclische Verbindung ist.

3. Verfahren zur Herstellung einer Nitrilverbindung gemäss Anspruch 1, wobei die organische Verbindung mindestens einen organischen Substituenten, ausgewählt aus der Gruppe bestehend aus Methyl, Ethyl, Propyl, Formyl, Acetyl, Hydroxymethyl und Methoxycarbonyl, in einem Molekül aufweist.

4. Verfahren zur Herstellung einer Nitrilverbindung gemäss Anspruch 1, wobei die organische Verbindung mindestens einen organischen Substituenten, ausgewählt aus der Gruppe bestehend aus Methyl, Ethyl, Propyl, Acetyl und Formyl, in einem Molekül aufweist.

5. Verfahren zur Herstellung einer Nitrilverbindung gemäss Anspruch 1, wobei die organische Verbindung mindestens eine, ausgewählt aus o-Xylol, m-Xylol und p-Xylol, ist.

6. Verfahren zur Herstellung einer Nitrilverbindung gemäss Anspruch 1, wobei die Gasphasenreaktion im Ammoxidierungs-Reaktionsschritt durch eine Festbett-Strömungsform durchgeführt wird.

7. Verfahren zur Herstellung einer Nitrilverbindung gemäss einem der Ansprüche 1 bis 6, wobei der im Ammoxidierungskatalysator enthaltene Diamant einen Teilchendurchmesser von grösser 0,001 bis 10 µm, gemessen mittels einer bestehenden Teilchengrössenmessvorrichtung vom Laserdispersionstyp, aufweist.

8. Verfahren zur Herstellung einer Nitrilverbindung gemäss einem der Ansprüche 1 bis 6, wobei der Ammoxidierungskatalysator ferner mindestens ein Oxid, ausgewählt aus der Gruppe bestehend aus Chromoxid, Molybdänoxid, Wolframoxid, Zirkoniumoxid, Eisenoxid, Kobaltoxid, Nickeloxid, Nioboxid, Boroxid, Aluminiumoxid, Germaniumoxid, Phosphoroxid, Antimonoxid und Wismutoxid, aufweist.

9. Verfahren zur Herstellung einer Nitrilverbindung gemäss einem der Ansprüche 1 bis 6, wobei der Ammoxidierungskatalysator ferner mindestens ein Oxid, ausgewählt aus der Gruppe bestehend aus Chromoxid, Molybdänoxid, Eisenoxid, Boroxid und Phosphoroxid, aufweist.

10. Verfahren zur Herstellung einer Nitrilverbindung gemäss einem der Ansprüche 1 bis 6, wobei der Ammoxidierungskatalysator auf einem Träger geträgert ist, der für die Reaktion inert ist.

11. Verfahren zur Herstellung einer Nitrilverbindung gemäss Anspruch 10, wobei der Träger ein geformter Träger ist, der mindestens eines, ausgewählt aus der Gruppe bestehend aus Siliciumcarbid, Aluminiumoxid, Silica, Zirkonoxid, Steatit, Cordierit, Mullit, Keramik und Porzellan, umfasst.

12. Verfahren zur Herstellung einer Nitrilverbindung gemäss Anspruch 10, wobei die Menge des geträgerten Ammoxidierungskatalysators 0,1 bis 20 Gew.%, in bezug auf das Gesamtgewicht des Ammoxidierungskatalysators und des Trägers, beträgt.

## Revendications

1. Processus de production d'un composé nitrile comprenant une étape de réaction d'ammoxydation dans laquelle on fait réagir un composé organique en phase gazeuse avec un gaz mixte comprenant de l'ammoniac et de l'oxygène en présence d'un catalyseur d'ammoxydation comprenant l'oxyde de vanadium, l'oxyde de titane et du diamant, où la teneur en diamant est de 0,1 à 70% en poids sur la base du poids du catalyseur d'ammoxydation.

2. Processus de production d'un composé nitrile selon la revendication 1, dans lequel le composé organique est un composé carbocyclique ou un composé hétérocyclique.

3. Processus de production d'un composé nitrile selon la revendication 1, dans lequel le composé organique a au moins un substituant organique choisi dans le groupe constitué de méthyle, d'éthyle, de propyle, de formyle, d'acétyle, d'hydroxyméthyle et de méthoxycarbonyle dans une molécule.

4. Processus de production d'un composé nitrile selon la revendication 1, dans lequel le composé organique a au moins un substituant organique choisi dans le groupe constitué de méthyle, d'éthyle, de propyle, d'acétyle et de formyle dans une molécule.

5. Processus de production d'un composé nitrile selon la revendication 1, dans lequel le composé organique est au moins un élément choisi parmi l'o-xylène, le m-xylène et le p-xylène.

6. Processus de production d'un composé nitrile selon la revendication 1, dans lequel la réaction en phase gazeuse dans l'étape de réaction d'ammoxydation est réalisée selon une forme d'écoulement en lit fixe.

7. Processus de production d'un composé nitrile selon l'une des revendications 1 à 6, dans lequel le diamant présent dans le catalyseur d'ammoxydation a un diamètre de particules allant de plus de 0,001 à 10 µm, mesuré au moyen d'un équipement existant de mesure de taille de particules de type à dispersion laser.

8. Processus de production d'un composé nitrile selon l'une des revendications 1 à 6, dans lequel le catalyseur d'ammoxydation comprend en outre au moins un oxyde choisi dans le groupe constitué d'oxyde de chrome, d'oxyde de molybdène, d'oxyde de tungstène, d'oxyde de zirconium, d'oxyde de fer, d'oxyde de cobalt, d'oxyde de nickel, d'oxyde de niobium, d'oxyde de bore, d'oxyde d'aluminium, d'oxyde de germanium, d'oxyde de phosphore, d'oxyde d'antimoine et d'oxyde de bismuth.

9. Processus de production d'un composé nitrile selon l'une des revendications 1 à 6, dans lequel le catalyseur d'ammoxydation comprend en outre au moins un oxyde choisi dans le groupe constitué d'oxyde de chrome, d'oxyde de molybdène, d'oxyde de fer, d'oxyde de bore et d'oxyde de phosphore.

10. Processus de production d'un composé nitrile selon l'une des revendications 1 à 6, dans lequel le catalyseur d'ammoxydation est supporté sur un support inerte vis-à-vis de la réaction.

11. Processus de production d'un composé nitrile selon la revendication 10, dans lequel le support est un support moulé comprenant au moins un élément choisi dans le groupe constitué de carbure de silicium, d'alumine, de silice, de zircone, de stéatite, de cordiérite, de mullite, de céramique et de porcelaines.

12. Processus de production d'un composé nitrile selon la revendication 10, dans lequel une quantité du catalyseur d'ammoxydation supporté est de 0,1 à 20% en poids sur la base d'un poids total du catalyseur d'ammoxydation et du support.
